(19) Europäisches Patentamt
European Patent Office
Office européen des brevets

(11) **EP 2 290 102 A1**

(12) **EUROPEAN PATENT APPLICATION**

(43) Date of publication:
**02.03.2011 Bulletin 2011/09**

(51) Int Cl.:
**C12Q 1/68** *(2006.01)*

(21) Application number: **09382108.0**

(22) Date of filing: **08.07.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicant: **Administración General De La Communidad Autónoma De Euskadi**
**01010 Vitoria-Gateiz (ES)**

(72) Inventors:
- **Otaegui Bichot, David**
  **20014 San Sebastián (ES)**
- **López de Munain Arregi, Adolfo**
  **20014 San Sebastián (ES)**

- **Olascoaga Urtaza, Javier**
  **20014 San Sebastián (ES)**
- **Calvo Molinos, Borja**
  **48940 Leioa, Bizkaia (ES)**
- **Armañanzas Arnedillo, Rubén**
  **48940 Leioa, Bizkaia (ES)**
- **Inza Cano, Iñaki**
  **48940 Leioa, Bizkaia (ES)**
- **Lozano Alonso, José Antonio**
  **48940 Leioa, Bizkaia (ES)**

(74) Representative: **ABG Patentes, S.L.**
**Avenida de Burgos 16D**
**Edificio Euromor**
**28036 Madrid (ES)**

(54) **Methods for the diagnosis of multiple sclerosis based on its microRNA expression profiling**

(57) The invention relates, in general, to a method for the diagnosis of multiple sclerosis based on determining amounts of one or more micro-RNAs correlated with multiple sclerosis in a biological sample from a subject.

FIG. 1

EP 2 290 102 A1

**SEQUENCE LISTING**

<110>  Administración General de la Comunidad Autónoma de Euskadi

<120>  METHODS FOR THE DIAGNOSIS OF MULTIPLE SCLEROSIS BASED ON ITS
       MICRORNA EXPRESSION PROFILING

<130>  P4659EP00

<160>  8

<170>  PatentIn version 3.5

<210>  1
<211>  22
<212>  RNA
<213>  Artificial Sequence

<220>
<223>  hsa-mir-18b miRNA

<400>  1
uaaggugcau cuagugcagu ua                                                22


<210>  2
<211>  22
<212>  RNA
<213>  Artificial Sequence

<220>
<223>  hsa-mir-493 miRNA

<400>  2
uuguacaugg uaggcuuuca uu                                                22


<210>  3
<211>  20
<212>  RNA
<213>  Artificial Sequence

<220>
<223>  hsa-mir-599 miRNA

<400>  3
guugugucag uuuaucaaac                                                   20


<210>  4
<211>  22
<212>  RNA
<213>  Artificial Sequence

<220>
<223>  hsa-mir-96 miRNA

<400>  4
uuuggcacua gcacauuuuu gc                                                22

```
<210>   5
<211>   22
<212>   RNA
<213>   Artificial Sequence

<220>
<223>   hsa-mir-148a miRNA

<400>   5
ucagugcacu acagaacuuu gu                                                    22


<210>   6
<211>   22
<212>   RNA
<213>   Artificial Sequence

<220>
<223>   hsa-mir-184 miRNA

<400>   6
uggacggaga acugauaagg gu                                                    22


<210>   7
<211>   22
<212>   RNA
<213>   Artificial Sequence

<220>
<223>   hsa-mir-30a-5p miRNA

<400>   7
uguaaacauc cucgacugga ag                                                    22


<210>   8
<211>   21
<212>   RNA
<213>   Artificial Sequence

<220>
<223>   hsa-mir-193 miRNA

<400>   8
aacuggccua caaaguccca g                                                     21
```

Description

FIELD OF THE INVENTION

The invention relates, in general, to methods for diagnosis of multiple sclerosis. In particular, the invention relates to diagnostic methods based on determining amounts of one or more micro-RNAs correlated with multiple sclerosis in a biological sample from a subject.

BACKGROUND OF THE INVENTION

Multiple sclerosis (MS) is an autoimmune demyelinating disease of the central nervous system (CNS). It begins most commonly during late adolescence, young adulthood, or mid-life, and it is one of the most incapacitating diseases in this age range.

MS causes attacks of neurological dysfunction (loss of vision, difficulty in walking or moving a limb, vertigo, loss of sensation) or progressive dysfunction in these same areas. These "attacks", also known as relapses, typically last for a few days, and resolve spontaneously. However, patients may not always completely recover from an attack and are sometimes left with a disability. Although most patients experience attacks with little or no progressive disability, approximately 10-15% have progressive symptoms from onset, called primary progressive forms. Furthermore, more than 80% of patients will ultimately develop progressive symptoms after a prolonged period of exacerbations, usually after 10-20 years.

Etiologically, MS is a complex disease in which both genetic and environmental factors play a role. The genetics of MS are also complex without a clear inheritance pattern. The most relevant candidate genomic region is the HLA system [Oksenberg, J.R. & Barcellos, L.F. Multiple sclerosis genetics: leaving no stone unturned. Genes Immun. 6, 375-387 (2005)], although several other genes are currently being described as important risk factors involved in MS, as for example IL2RA [Alcina, A. et al. IL2RA/CD25 gene polymorphisms: uneven association with multiple sclerosis (MS) and type 1 diabetes (T1D). PLoS. ONE. 4, e4137 (2009)] or IL7R genes [Gregory, S.G. et al. Interleukin 7 receptor alpha chain (IL7R) shows allelic and functional association with multiple sclerosis. Nat. Genet. 39, 1083-1091 (2007)].

Gene expression profiling has been a useful tool to provide information about the molecular pathways involved in MS pathogenesis [Achiron, A., Gurevich, M., Friedman, N., Kaminski, N., & Mandel, M. Blood transcriptional signatures of multiple sclerosis: unique gene expression of disease activity. Ann. Neurol. 55, 410-417 (2004); Baranzini, S.E. et al. Transcription-based prediction of response to IFNbeta using supervised computational methods. PLoS. Biol. 3, e2 (2005); Ramanathan, M. et al. In vivo gene expression revealed by cDNA arrays: the pattern in relapsing-remitting multiple sclerosis patients compared with normal subjects. J. Neuroimmunol. 116, 213-219 (2001)]. Several new studies have identified different expression patterns between relapses and remission [Otaegui, D. et al. Increased transcriptional activity of milk-related genes following the active phase of experimental autoimmune encephalomyelitis and multiple sclerosis. J. Immunol. 179, 4074-4082 (2007); Satoh, J., Misawa, T., Tabunoki, H., & Yamamura, T. Molecular network analysis of T-cell transcriptome suggests aberrant regulation of gene expression by NF-kappaB as a biomarker for relapse of multiple sclerosis. Dis. Markers 25, 27-35 (2008)] showing that this clinical differentiation of two states of the disease also has a molecular correlation.

Recently a new expression and protein synthesis modulator has been identified: the small non-coding RNA molecules (microRNA or miRNA). These miRNA are single-stranded RNA molecules of about 20-25 nucleotides (nt) encoded by nuclear genes (70-150 nt) and highly conserved among species. These genes are not translated into proteins but are processed from primary transcripts (called pri-miRNA) to short stem-loop structures called pre-miRNA and finally to functional miRNA. The expression pattern of miRNA varies over time and between tissues. These mature miRNA molecules are partially complementary to one or more mRNA sequences and they function through sequence-specific down-regulation of their target mRNA via mRNA degradation or inhibition of translation [Bartel, D.P. MicroRNAs: genomics, biogenesis, mechanism, and function. Cell 116, 281-297 (2004)]. Initial estimates suggested there were more than 500 validated human miRNA [Griffiths-Jones, S., et al. miRBase: microRNA sequences, targets and gene nomenclature. Nucleic Acids Res. 34, D140-D144 (2006); Griffiths-Jones,S. The microRNA Registry. Nucleic Acids Res. 32, D109-D111 (2004)], although in the public database around 700 were proposed in October 2008 (www.microrna.sanger.ac.uk).

It has been predicted that miRNA may regulate around 30% of all cellular mRNA so they should play a critical role in virtually all cellular functions [Lewis, B.P., Burge, C.B., & Bartel, D.P. Conserved seed pairing, often flanked by adenosines, indicates that thousands of human genes are microRNA targets. Cell 120, 15-20 (2005)].

Although misregulation of miRNA expression has been characterized mostly in cancer, it has recently been studied in many other diseases. Thus, miRNA has been proposed as a regulator of immune cell development [Baltimore, D., Boldin, M.P., O'Connell, R.M., Rao, D.S., & Taganov, K.D. MicroRNAs: new regulators of immune cell development and function. Nat. Immunol. 9, 839-845 (2008)], playing a role in the inflammatory response [O'Connell, R.M., Taganov, K.D., Boldin, M.P., Cheng, G., & Baltimore, D. MicroRNA-155 is induced during the macrophage inflammatory response. Proc. Natl. Acad. Sci. U. S. A. 104, 1604-1609 (2007)], and as a key player in the pathogenesis of neurodegenerative diseases [Nelson, P.T., Wang, W.X., & Rajeev, B.W. MicroRNAs (miRNAs) in neurodegenerative diseases. Brain Pathol. 18, 130-138 (2008)]. Further, WO 2008/147974 discloses the role of some miRNAs (e.g., hsa-mir-493) in the pathogenesis of asthma; WO 2009/009457 discloses methods of diagnosis and prognosis of Alzheimer's disease in subjects by measuring amounts of one or more miRNAs (e.g, hsa-mir-186 and hsa-mir-493-3p); WO 2008/153692 discloses the role of hsa-mir-186 and hsa-mir-96 in brain tumors and hsa-mir-30a-5p is up-regulated by wt-p53 in human colon cancer cell lines HCT-116 (wt-p53) [Yaguang X et al., Clin. Cancer Res 2006; 12(7):2014-2024].

Currently MS is diagnosed by clinical observation following the Polman Criteria [Polman CH, Reingold SC, Edan G, Filippi M, Hartung HP, Kappos L, Lublin FD, Metz LM, McFarland HF, O'Connor PW, Sandberg-Wollheim M, Thompson AJ, Weinshenker BG, Wolinsky JS. Diagnostic criteria for multiple sclerosis: 2005 revisions to the "McDonald Criteria Ann Neurol. 2005 Dec;58(6):840-6]. Basically these criteria are focussed in the dissemination of lesions in both time and space combining with other clinical and paraclinical diagnostic methods. Time is one of the drawbacks of said criteria since it is one of the parameters used in the diagnosis and a patient could need a period between six months to one year to be diagnosed with MS. In this scenario an objective tool that could help in the diagnosis of MS would be very useful, reducing the time, and, maybe, the cost of the diagnostic.

On the other hand, the definition of relapse is some times difficult because some relapses, i.e. sensorial symptoms, could have no neurological origin. Moreover, some relapses are subclinical so they are manifested only in the magnetic resonance imaging (MRI). Thus, a biological marker of the relapse could be useful not only to characterize the relapse but also to evaluate the treatment.

## SUMMARY OF THE INVENTION

The present invention is based upon the discovery that some miRNAs are expressed at different levels not only in subjects afflicted with MS (i.e., MS patients) versus controls but also in the relapse status of the disease. These observations provide a basis for the concept that assays of microRNA levels may be used in diagnosing MS and in the post-therapy monitoring of patients. In particular, a comparison can be made between the levels of miRNA of a test subject and in that of one or more control subjects. Comparisons can be made directly. Measurements of miRNA levels may be carried out using singleplex (involving one set of primers) or multiplex (involving more than one set of primers) qRT-PCR.

Inventors have studied the expression pattern of 364 miRNAs in peripheral blood mononuclear cells (PBMC) obtained from multiple sclerosis patients in relapse status, in remitting status and healthy controls with the aim of understanding the regulatory mechanisms of said stages. Expression values were obtained by means of qPCR. The most relevant miRNA were validated in an independent set of samples. In order to determine the effect of the miRNA pattern, the expression of some predicted target genes was studied by qPCR. Gene interaction networks were constructed in order to obtain a system biology and multivariate view of the experimental data. The data analysis and later validation reveal that some miRNAs, e.g., hsa-mir-18b, hsa-mir-493 and hsa-mir-599, specially hsa-mir-18b and hsa-mir-599, may be relevant at the time of relapse and that other miRNAs, e.g., hsa-mir-96, hsa-mir-148a, hsa-mir-184, hsa-mir-30a-5p and hsa-mir-193, specially hsa-mir-96, may be involved in the remitting phenomena (Example 1).

## BRIEF DESCRIPTION OF DRAWINGS

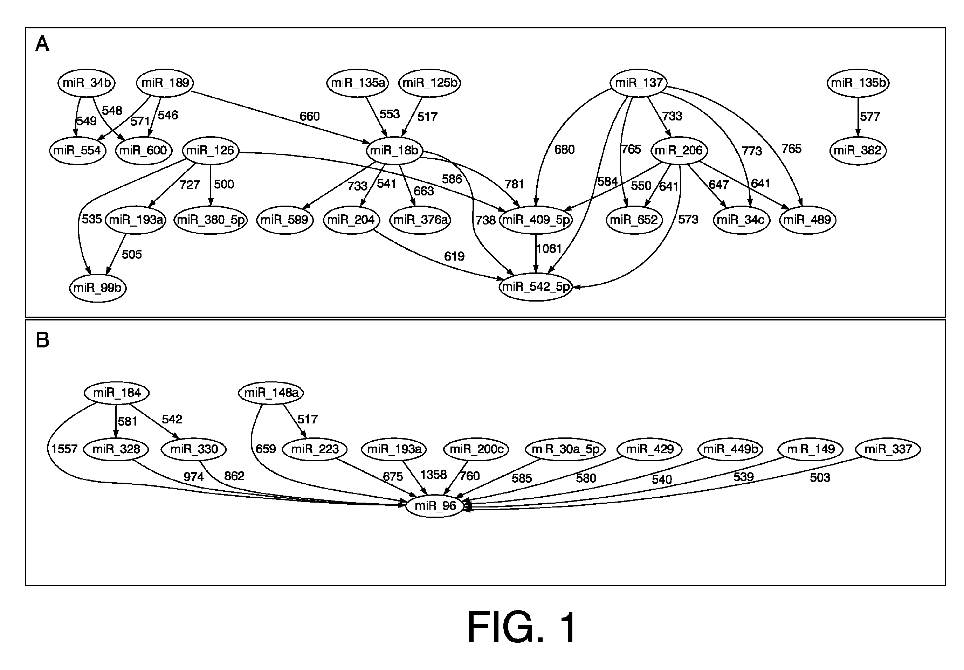

Figure 1 is a diagram depicting the gene interaction networks from the qPCR data. A: Relapse status versus remitting status. B: Remitting status versus controls.
Figure 2 are graphs showing data the percentage of the targets founded in the EAE experiment (A: EAE lymph node target genes; B: EAE spinal cord target genes). The found targets were grouped in up-regulated, down-regulated and equally regulated between the EAE model and the control. The data from the 8 selected miRNAs are shown (clear) as well as the data from the randomly selected 11 miRNAs (dark).

## DETAILED DESCRIPTION OF THE INVENTION

Definitions

A "microRNA"("miRNA') is a naturally occurring, small non-coding RNA that is about 17 to about 25 nucleotide (nt) in length in its biologically active form that negatively regulates mRNA translation on a sequence-specific manner. MicroRNAs (miRNAs) post-transcriptionally regulate gene expression by repressing target mRNA translation. It is thought that miRNAs function as negative regulators, i.e. greater amounts of a specific miRNAwill correlate with lower levels of target gene expression. There are three forms of miRNAs existing *in vivo,* primary miRNAs (pri-miRNAs), premature miRNAs (pre-miRNAs), and mature miRNAs. Primary miRNAs (pri-miRNAs) are expressed as stem-loop structured transcripts of about a few hundred bases to over 1 kilobase (kb). The pri-miRNA transcripts are cleaved in the nucleus by an RNase II endonuclease that cleaves both strands of the stem near the base of the stem loop. The cleavage product, the premature miRNA (pre-miRNA) is about 60 to about 110 nt long with a hairpin structure formed in a fold-back manner. Pre-miRNA is transported from the nucleus to the cytoplasm. Pre-miRNAs are processed further in the cytoplasm by another RNase II endonuclease to form miRNA duplexes. The miRNA duplex binds to the RNA-induced silencing complex (RISC), where the antisense strand is preferentially degraded and the sense strand mature miRNA directs RISC to its target site. It is the mature miRNA that is the biologically active form of the miRNA. Identified miRNAs are registered in the miRNA database miRBase (http://microma.sanger.ac.uk/). It is believed that the number of miRNAs could exceed 1,000, which constitute approximately 1-5% of the expressed genes. Bioinformatics analyses suggest that, on average, each miRNA may regulate about 200 genes. Therefore, as much as 30% to 50% of all human genes may be under miRNA control. Since miRNAs may play important roles in cell development, proliferation and apoptosis, their expression patterns are highly regulated; in fact, deregulation of miRNA function might contribute to many human diseases.

A "sample", as defined herein, is a small part of a subject, representative of the whole and may be constituted by a biopsy or a body fluid sample. Biopsies are small pieces of tissue and may be fresh, frozen or fixed, such as formalin-fixed and paraffin embedded (FFPE). Body fluid samples may be blood, plasma, serum, urine, sputum, cerebrospinal fluid, milk, or ductal fluid samples and may likewise be fresh, frozen or fixed. Samples may be removed surgically, by extraction i.e. by hypodermic or other types of needles, by microdissection or laser capture. The sample should contain any biological material suitable for detecting the desired biomarker (miRNA), thus, said sample should, advantageously comprise cell material from the subject. Thus, in a particular embodiment, the sample is a body fluid sample such as a blood sample; preferably, the sample comprises peripheral blood mononuclear cells (PBMC) from the subject.

A "reference sample", as used herein, means a sample obtained from subjects, preferably two or more subjects, known to be free of the disease (MS) or, alternatively, from the general population. The suitable reference expression levels of miRNAs can be determined by measuring the expression levels of said miRNAs in several suitable subjects, and such reference levels can be adjusted to specific subject populations. In a preferred embodiment, the reference sample is obtained from a pool of healthy subjects or from subjects without prior history of MS. The expression profile of the miRNAs in the reference sample can, preferably, be generated from a population of two or more subjects; for example, the population can comprise 3, 4, 5, 10, 15, 20, 30, 40, 50 or more subjects.

A "subject", as used herein, refers to a mammal, human or non-human, under observation, preferably a human being. The subject may be any subject, a subject predisposed of a disease (e.g., MS) or a subject suffering from said disease.

"Multiple sclerosis" ("MS") is a disease in which there are demyelination foci of several sizes throughout the white matter of the central nervous system (CNS), sometimes extending into the gray matter, giving rise weakness, incoordination, paresthesias, speech disorders, and visual complaints. MS is a disease of unknown etiology with a prolonged course involving many remissions and relapses. The term "multiple sclerosis" or "MS", as used herein, is meant to include benign multiple sclerosis (benign MS), relapsing-remitting multiple sclerosis (RRMS), secondary progressive multiple sclerosis (SPMS), primary progressive multiple sclerosis (PPMS), and progressive-relapsing multiple sclerosis (PRMS). These subtypes or forms of the disease (MS) may be distinguished from one another on the basis of the course of the disease, of the type of inflammation involved, and through the use of magnetic resonance imaging (MRI). Chronic progressive multiple sclerosis is a term used to collectively refer to SPMS, PPMS, and PRMS. The relapsing forms of multiple sclerosis are SPMS with superimposed relapses, RRMS, and PRMS.

As used herein, the expression "diagnosis" or "diagnosing" relates to methods by which the skilled artisan can estimate and even determine whether or not a subject is suffering from a given disease or condition. The skilled artisan often makes a diagnosis on the basis of one or more diagnostic indicators, such as for example a biomarker (e.g., a miRNA or a set of miRNAs), the amount (including presence or absence) of which is indicative of the presence, severity, or absence of the condition. It also relates to evaluating the probability according to which a subject suffers from a disease. As it will be understood by persons skilled in the art, such evaluation, although it is preferred that it is, normally may not be correct for 100% of the subjects to be diagnosed. The term, however, requires that a statistically significant part of the subjects can be identified as suffering from the disease or having a predisposition for it. The person skilled in the art can determine if a part is statistically significant without further delay by using several well known statistic evaluation tools, for example, determination of confidence intervals, determination of the p value, Student's t-test, Mann-Whitney test, etc. The details are in Dowdy and Wearden, Statistics for Research, John Wiley & Sons, New York 1983. The preferred confidence intervals are of at least 50%, at least 60%, at least 70%, at least 80%, at least 90%, at least 95%. The p values are preferably 0.2, 0.1, 0.05.

Along with diagnosis, clinical disease prognosis is also an area of great concern and interest. It is important to know the stage and rapidity of advancement of the MS in order to plan the most effective therapy. If a more accurate prognosis can be made, appropriate therapy, and in some instances less severe therapy for the patient can be chosen. Measurement of miRNA levels disclosed herein can be useful in order to categorize subjects according to advancement of MS who will benefit from particular therapies and differentiate from other subjects where alternative or additional therapies can be more appropriate.

Further, the expression "method of diagnosing" as used herein relates to a method that may essentially consist of the steps mentioned below (or may include additional steps. However, it must be understood that the method, in a preferred embodiment, is a method that is carried out *in vitro,* i.e., it is not carried out in the human or animal body.

The terms "PCR reaction", "PCR amplification", "PCR", "pre-PCR", "Q-PCR", "qPCR", "real-time quantitative PCR" and "real-time quantitative RT-PCR" are interchangeable terms used to mean the use of a nucleic acid amplification system, which multiplies the target nucleic acids being detected. Examples of such systems include the polymerase chain reaction (PCR) system and the ligase chain reaction (LCR) system. Other methods recently described and known to the person skilled in the art include the nucleic acid sequence based amplification and Q Beta Replicase systems. The products formed by said amplification reaction may or may not be monitored in real time or only after the reaction as an end-point measurement.

Diagnostic methods of the invention

The inventors have identified a set of specific miRNA which are differentially expressed in MS patients with respect to reference (non-MS) samples.

Accordingly, in an aspect, the invention relates to a method of diagnosing Multiple Sclerosis (MS) in a subject, hereinafter referred to as the method of the invention, which comprises comparing:

a) the level of expression of a miRNA selected from the group consisting of hsa-mir-18b, hsa-mir-493, hsa-mir-599, hsa-mir-96, hsa-mir-148a, hsa-mir-184, hsa-mir-30a-5p, hsa-mir-193 and combinations thereof, in a sample from said subject; and
b) the normal level of expression of said miRNA(s) in a reference sample,

wherein a statistically significant increase in the level of expression of said miRNA in the subject sample with respect to the normal level of said miRNA(s) in the reference sample is indicative that the subject is afflicted with MS.

Briefly, the method of the invention involves obtaining a sample from the subject (i.e., a test sample) and assaying said sample to determine the level of expression (i.e., the concentration or amount) of one or more miRNAs selected from the group consisting of hsa-mir-18b, hsa-mir-493, hsa-mir-599, hsa-mir-96, hsa-mir-148a, hsa-mir-184, hsa-mir-30a-5p, hsa-mir-193 and combinations thereof. The expression level of said miRNAs can be determined by any suitable method, e.g., multiplex and/or singleplex real-time RT-PCR, including quantitative real time reverse transcriptase PCR (qRT-PCR); single-molecule detection; bead-based flow cytometric methods; assays using arrays of nucleic acids; etc.

The sample can be obtained by any conventional method depending on the sample to be analyzed. In a particular embodiment, the sample is a body fluid sample such as a blood sample, e.g., a sample comprising PBMC from the subject. Before analyzing the sample, it will often be desirable to perform one or more sample preparation operations upon the sample. Typically, these sample preparation operations include manipulations such as concentration, suspension, extraction of intracellular material, e.g., nucleic acids from tissue/whole cell samples and the like, amplification of nucleic acids, fragmentation, transcription, labeling and/or extension reactions. Nucleic acids, especially RNA and specifically miRNA can be isolated using any techniques known in the art. There are two main methods for isolating RNA: (i) phenol-based extraction and (ii) silica matrix or glass fiber filter (GFF)-based binding. Phenol-based reagents contain a combination of denaturants and RNase inhibitors for cell and tissue disruption and subsequent separation of RNA from contaminants. Phenol-based isolation procedures can recover RNA species in the 10-200-nucleotide range e.g., miRNAs, 5S rRNA, 5.8S rRNA, and U1 snRNA. If a sample of "total" RNA was purified by the popular silica matrix column or GFF procedure, it may be depleted in small RNAs. Extraction procedures such as those using Trizol or TriReagent, however will purify all RNAs, large and small, and are the recommended methods for isolating total RNA from biological samples that will contain miRNAs/siRNAs. Any method required for the processing of a sample prior to quantifying the level of the miRNAs according to the method of the invention falls within the scope of the present invention. These methods are typically well known by a person skilled in the art.

According to the method of the invention, the specific miRNAs that are tested for include one or more of the following: hsa-mir-18b, hsa-mir-493, hsa-mir-599, hsa-mir-96, hsa-mir-148a, hsa-mir-184, hsa-mir-30a-5p, and hsa-mir-193. The designations provided are standard in the art and are associated with specific sequences that can be found at the miRNA registry (http://microrna.sanger.ac.uk/sequences/). In all cases, they refer to human sequences as shown in Table 1.

**Table 1**

| MicroRNA sequences | | |
| --- | --- | --- |
| **MicroRNA** | **Sequence** | **SEQ ID NO:** |
| hsa-miR-18b | UAAGGUGCAUCUAGUGCAGUUA | 1 |
| hsa-mir-493 | UUGUACAUGGUAGGCUUUCAUU | 2 |
| hsa-mir-599 | GUUGUGUCAGUUUAUCAAAC | 3 |
| hsa-mir-96 | UUUGGCACUAGCACAUUUUUGC | 4 |
| hsa-mir-148a | UCAGUGCACUACAGAACUUUGU | 5 |
| hsa-mir-184 | UGGACGGAGAACUGAUAAGGGU | 6 |
| hsa-mir-30a-5p | UGUAAACAUCCUCGACUGGAAG | 7 |
| hsa-mir-193 | AACUGGCCUACAAAGUCCCAG | 8 |

In some cases, there could be additional family members of said miRNAs (Table 1) that are recognized in the art and which should be considered equivalents of the specific sequences listed herein. Although all sequences are shown as RNA sequences, it will be understood that, when referring to hybridizations or other assays, corresponding DNA sequences can be used as well. For example, RNA sequences may be reverse transcribed and amplified using the polymerase chain reaction (PCR) in order to facilitate detection. In those cases, it will actually be DNA and not RNA that is directly quantified. It will also be understood that the complement of the reverse transcribed DNA sequences can be analyzed instead of the sequence itself. In this context, the term "complement" refers to an oligonucleotide that has an exactly complementary sequence, i.e. for each adenine (A) there is a thymine (T), etc. Although assays may be performed for the miRNAs individually, it is generally preferable to assay several miRNAs.

The level of expression of said miRNAs (Table 1) can be determined by any suitable method including generic methods for the detection and quantification of nucleic acids especially RNA, optimized methods for the detection and quantification of small RNA species, as both mature and precursor miRNAs fall into this category as well as specially designed methods for the detection and quantification of miRNA species. Illustrative, non-limitative, examples of methods which can be used to facilitate the testing of multiple miRNAs include:

> 1) multiplex and/or singleplex real-time RT-PCR (reagents available from, e.g., Applied Biosystems and System Biosciences (SBI)), including quantitative real time reverse transcriptase PCR (qRT-PCR) as described in US 5,928,907 and US 6,015,674;
> 2) single-molecule detection as described by Neely, et al., Nat. Methods. 3(1):41-6 (2006) and in US 6,355,420; US 6,916,661; and US 6,632,526;
> 3) bead-based flow cytometric methods as described by Lu, et al., Nature 435:7043 (2005) and in US 6,524,793; and
> 4) assays using arrays of nucleic acids as described by Nelson, et al., Nat. Methods 1(2):155-61 (2004); Wu, et al., RNA 13(1):151-9 (2007) and in US 6,057,134; US 6,891,032; US 7,122,303; US 6,458,583; US 6,465,183; US 6,461,816; US 6,458,583; US 7,026,124; US 7,052,841; US 7,060,809; US 6,436,640; and US 7,060,809.

Microarrays can also be prepared in which oligonucleotides having complementary sequences (or oligonucleotides with sequences matching the miRNAs themselves) are immobilized on the surface of a solid support. Materials that can be used as supports include membranes, dishes or slides made of glass or plastic. At least 1, and, preferably, 2, 3, 4, 5, 6, 7 or 8 of the miRNAs described above (Table 1) should be recognized by the immobilized oligonucleotides, with each different oligonucleotide occupying a distinct and known position on the support. Microarrays of this type may be made by using methodology well known in the art or appropriate miRNA arrays can be purchased commercially, e.g., from Ambion, Agilent or Exiqon. MiRNA can then be isolated from the sample of the subject (e.g., using Ambion's Leucolock kit (AM1923)), amplified by using, e.g., the polymerase chain reaction (PCR), and analyzed by hybridizations performed under stringent conditions. The term "stringent conditions" indicates conditions that essentially only permit hybridization to occur with the exact complementary sequence of the immobilized oligonucleotide. In general, these hybridizations are performed in buffers of about neutral pH containing 0.1-0.5 NaCl and at a temperature of between 37°C to 50°C. It is also possible to carry out incubations under conditions of low stringency and then to use high stringency wash conditions to cause the dissociation of hybridized sequences that are not exact matches.

One way to carry out microarray assays would involve amplifying miRNA in the presence of a detectable label, e.g., a nucleotide bound to a dye or other marker and present in a PCR primer. Thus, a population of labeled cDNAs may be obtained that can be used directly in hybridizations with oligonucleotides immobilized on a microarray plate or slide. After hybridizations are completed, plates may be analyzed using an automated reader to determine the amount of label associated with each immobilized sequence, which, in turn, reflects the abundance of the hybridized sequence in the original miRNA population. Many variations of this basic procedure have been described in the art and are compatible with the present invention.

In a particular embodiment, the expression level of one or more of the following miRNA: hsa-mir-18b, hsa-mir-493, hsa-mir-599, hsa-mir-96, hsa-mir-148a, hsa-mir-184, hsa-mir-30a-5p, and hsa-mir-193, is determined by real-time quantitative RT-PCR (qRT-PCT), a modification of the polymerase chain reaction (PCR) used to rapidly measure the quantity of a product of the PCR. It is preferably done in real-time, thus it is an indirect method for quantitatively measuring starting amounts of DNA, complementary DNA or RNA. This is commonly used for the purpose of determining whether a genetic sequence is present or not, and if it is present the number of copies in the sample. Like other forms of PCR, the process is used to amplify DNA samples, using thermal cycling and a thermostable DNA polymerase. The three commonly used methods of quantitative polymerase chain reaction are through agarose gel electrophoresis, the use of SYBR Green (a double stranded DNA dye), and the fluorescent reporter probe. The latter two of these three can be analysed in real-time, constituting real-time polymerase chain reaction method.

Using SYBR Green dye gives results in real time. A DNA binding dye binds all newly synthesized double stranded (ds)DNA and an increase in fluorescence intensity is measured, thus allowing initial concentrations to be determined. However, SYBR Green will label all dsDNA including any unexpected PCR products as well as primer dimers, leading to potential complications and artefacts. The reaction is prepared as usual, with the addition of fluorescent dsDNA dye. The reaction is run, and the levels of fluorescence are monitored; the dye only fluoresces when bound to the dsDNA. With reference to a standard sample or a standard curve, the dsDNA concentration in the PCR can be determined.

The fluorescent reporter probe method is the most accurate and most reliable of the methods. It uses a sequence-specific nucleic acid based probe so as to only quantify the probe sequence and not all double stranded DNA. It is commonly carried out with DNA based probes with a fluorescent reporter and a quencher held in adjacent positions, so-called dual- labelled probes. The close proximity of the reporter to the quencher prevents its fluorescence; it is only on the breakdown of the probe that the fluorescence is detected. This process depends on the 5' to 3' exonuclease activity of the polymerase involved. The real-time quantitative PCR reaction is prepared with the addition of the dual-labelled probe. On denaturation of the double-stranded DNA template, the probe is able to bind to its complementary sequence in the region of interest of the template DNA (as the primers will too). When the PCR reaction mixture is heated to activate the polymerase, the polymerase starts synthesizing the complementary strand to the primed single stranded template DNA. As the polymerisation continues it reaches the probe bound to its complementary sequence, which is then hydrolysed due to the 5'-3' exonuclease activity of the polymerase thereby separating the fluorescent reporter and the quencher molecules. This results in an increase in fluorescence, which is detected. During thermal cycling of the real-time PCR reaction, the increase in fluorescence, as released from the hydrolysed dual-labelled probe in each PCR cycle is monitored, which allows accurate determination of the final, and so initial, quantities of DNA.

Any method of PCR that can determine the expression of a nucleic acid molecule as defined herein falls within the scope of the present invention. A preferred embodiment of the present invention regards the real-time quantitative RT-PCR method, based on the use of either SYBR Green dye or a dual-labelled probe for the detection and quantification of nucleic acids according to the herein described. A more preferred embodiment of the present invention regards the methods of real-time quantitative RT-PCR for the expression profiling of miRNAs in MS.

The results obtained are compared with those obtained using reference samples. Nevertheless, it will be understood that it is not absolutely essential that an actual reference sample be run at the same time that assays are being performed on a test sample. Once reference (normal) levels of the miRNAs have been established, said levels can provide a basis for comparison without the need to rerun a new reference sample with each assay. The comparison between the test and reference samples provides a basis for a conclusion as to whether a subject has MS. In general, the greater the difference between the test sample and the reference sample, the stronger the indication for the presence of MS. Thus, the expression level of the MS diagnostic miRNA (Table 1) once determined in the test sample is compared with the expression level of said miRNAs in a reference sample; at a minimum, a difference of about 10% (i.e., a 1.1 fold change) should be seen to conclude that a disease (MS) is present with higher differences, e.g., about 15%, about 20%, about 25%, about 30%, about 50% (i.e., a 1.5 fold change), about 60%, about 75%, about 100% (i.e., a 2 fold change), or more (e.g., about 150%), being more conclusive.

The above-identified miRNAs (Table 1) are particularly useful in the diagnosis of MS.

In a particular embodiment, said miRNAs are one or more of hsa-mir-18b, hsa-mir-493 and hsa-mir-599; said miRNAs showing a differential expression in subjects afflicted with MS experiencing a relapse (i.e., increased levels of said miRNAs are indicative of the presence of MS in a relapse status); most preferably, said miRNA is selected from the group consisting of hsa-mir-18b, hsa-mir-599 and combinations thererof.

In another particular embodiment, said miRNAs are one or more of hsa-mir-96, hsa-mir-148a, hsa-mir-184, hsa-mir-30a-5p, and hsa-mir-193, said miRNAs showing a differential expression in subjects afflicted with MS experiencing a remission (i.e., increased levels of said miRNAs are indicative of the presence of MS in a remission status); most preferably, said miRNA is hsa-mir-96.

Therefore, in another aspect, the invention relates to a method for assessing if a subject afflicted with Multiple Sclerosis (MS) is experiencing a relapse, which comprises comparing:

a) the level of expression of a miRNA selected from the group consisting of hsa-mir-18b, hsa-mir-493, hsa-mir-599, and combinations thereof, in a test sample from said subject; and
b) the normal level of expression of said miRNA(s) in a reference sample,

wherein a statistically significant increase in the level of expression of said miRNA(s) in the subject sample with respect to the normal level of said miRNA(s) in the reference sample is indicative that the subject afflicted with MS is experiencing a relapse.

"Relapse" could be defined as the appearance of new symptoms or the aggravation of old ones, lasting at least twenty-four hours (synonymous with attack, relapse, flare-up, or worsening), usually associated with inflammation and demyelination in the brain or spinal cord. To be a true exacerbation, the attack must last at least 24 hours and be separated from the previous attack by at least 30 days. Most exacerbations last from a few days to several weeks or even months.

The particulars of the samples, both the test and the reference samples, have been previously mentioned in connection with the method of the invention. The expression levels of said miRNAs (hsa-mir-18b, hsa-mir-493, hsa-mir-599, and combinations thereof) can be determined as discussed in connection with the method of the invention as well as what should be considered as a statistically significant increase.

Further, in another aspect, the invention relates to a method for assessing if a subject afflicted with Multiple Sclerosis (MS) is experiencing a remission, which comprises comparing:

a) the level of expression of a miRNA selected from the group consisting of hsa-mir-96, hsa-mir-148a, hsa-mir-184, has-mir-30a-5p, hsa-mir-193, and combinations thereof, in a test sample from said subject; and
b) the normal level of expression of said miRNA(s) in a reference sample,

wherein a statistically significant increase in the level of expression of said miRNA(s) in the subject sample with respect to the normal level of said miRNA(s) in the reference sample is indicative that the subject afflicted with MS is experiencing a remission.

"Remission" could be defined as a lessening in the severity of symptoms or their temporary disappearance during the course of the illness. It also refers to a patient with RR-MS without clinical symptoms of a relapse.

The particulars of the samples, both the test and the reference samples, have been previously mentioned in connection with the method of the invention. The expression levels of said miRNAs (hsa-mir-96, hsa-mir-148a, hsa-mir-184, hsa-mir-30a-5p, hsa-mir-193, and combinations thereof) can be determined as discussed in connection with the method of the invention as well as what should be considered as a statistically significant increase.

Further, in another aspect, the invention relates to a method for assessing if a subject afflicted with Multiple Sclerosis (MS) is experiencing a remission, which comprises comparing:

a) the level of expression of a miRNA selected from the group consisting of hsa-mir-18b, hsa-mir-493, hsa-mir-599, and combinations thereof, in a test sample from said subject; and
b) the level of expression of said miRNA(s) in a control sample, said control sample being a sample from the same subject under analysis obtained during a period in which said subject afflicted with MS under analysis is experiencing a relapse,

wherein a statistically significant decrease in the level of expression of said miRNA(s) in the subject sample with respect to the level of said miRNA(s) in the control sample is indicative that the subject afflicted with MS is experiencing a remission.

The particulars of the test samples have been previously mentioned in connection with the method of the invention. The expression levels of said miRNAs (hsa-mir-18b, hsa-mir-493, hsa-mir-599, and combinations thereof) can be determined as discussed in connection with the method of the invention. According to this aspect of the invention, the level of expression of said miRNA(s) in a test sample from the subject under analysis is compared to the level of expression of said miRNA(s) in a control sample, said "control" sample being a sample from the same subject under analysis obtained during a period in which said subject afflicted with MS under analysis is experiencing a relapse. If a subject afflicted with MS is experiencing a relapse can be assessed as mentioned above. The comparison between the test and control samples provides a basis for a conclusion as to whether a subject afflicted with MS is experiencing a remission. A difference of about 10% between the expression level of said miRNA in the test sample with respect to the control sample should be seen to conclude that a subject afflicted with MS is experiencing a remission, with higher differences, e.g. about 15%, about 20%, about 25%, about 30%, about 50% (i.e., a 1.5 fold change), about 60%, about 75%, or more, being more conclusive.

Further, in another aspect, the invention relates to a method for assessing if a subject afflicted with Multiple Sclerosis (MS) is experiencing a relapse, which comprises comparing:

a) the level of expression of a miRNA selected from the group consisting of hsa-mir-96, hsa-mir-148a, hsa-mir-184, hsa-mir-30a-5p, hsa-mir-193, and combinations thereof, in a test sample from said subject; and

b) the level of expression of said miRNA(s) in a control sample, said control sample being a sample from the same subject under analysis obtained during a period in which said subject afflicted with MS under analysis is experiencing a remission,

wherein a statistically significant decrease in the level of expression of said miRNA(s) in the subject sample with respect to the level of said miRNA(s) in the control sample is indicative that the subject afflicted with MS is experiencing a relapse.

The particulars of the test samples have been previously mentioned in connection with the method of the invention. The expression levels of said miRNAs (hsa-mir-96, hsa-mir-148a, hsa-mir-184, hsa-mir-30a-5p, hsa-mir-193, and their combinations) can be determined as discussed in connection with the method of the invention. According to this aspect of the invention, the level of expression of said miRNA(s) in a test sample from the subject under analysis is compared to the level of expression of said miRNA(s) in a control sample, said "control" sample being a sample from the same subject under analysis obtained during a period in which said subject afflicted with MS under analysis is experiencing a remission. If a subject afflicted with MS is experiencing a remission can be assessed as mentioned above. The comparison between the test and control samples provides a basis for a conclusion as to whether a subject afflicted with MS is experiencing a relapse. A difference of about 10% between the expression level of said miRNA in the test sample with respect to the control sample should be seen to conclude that a subject afflicted with MS is experiencing a relapse, with higher differences, e.g., about 15%, about 20%, about 25%, about 30%, about 50% (i.e., a 1.5 fold change), about 60%, about 75%, or more, being more conclusive.

Additionally, the teachings of the invention can be used to design a therapy to the subject afflicted with MS. Thus, in a further aspect, the invention relates to a method of designing a therapy for a subject afflicted with Multiple Sclerosis (MS), which comprises:

- determining if a subject afflicted with MS is experiencing a relapse, and selecting a drug suitable for treatment of MS in a relapse status; or, alternatively,
- determining if a subject afflicted with MS is experiencing a remission, and selecting a drug suitable for treatment of MS in a remission status.

If a subject afflicted with MS is experiencing a relapse can be determined according to the above mentioned method. Illustrative, non-limitative examples of drugs suitable for treatment of MS in a relapse status include corticosteroids, etc. In fact, during symptomatic attacks, administration of high doses of intravenous corticosteroids, such as methylprednisolone, is the routine therapy for acute relapses.

If a subject afflicted with MS is experiencing a remission can be determined according to the above mentioned method. Illustrative, non-limitative examples of drugs suitable for treatment of MS include interferon beta-1a (e.g., marketed under trade names *Avonex, CinnoVex, ReciGen* or *Rebif*) and one of interferon beta-1b (marketed in the US under trade name *Betaseron,* and in Europe and Japan as *Betaferon),* glatiramer acetate *(Copaxone),* mitoxantrone, and natalizumab (marketed under trade name *Tysabri*).

The following Example has been included to illustrate a mode of the presently-disclosed subject matter. In light of the present disclosure and the general level of skill in the art, those of skill will appreciate that the following Example is intended to be exemplary only and that numerous changes, modifications, and alterations can be employed without departing from the scope of the presently-disclosed subject matter.

## EXAMPLE 1

### Differential miRNA expression in PBMC from MS patients

Inventors have studied the expression pattern of 364 microRNA in peripheral blood mononuclear cells (PBMC) obtained from multiple sclerosis patients in relapse status, in remitting status and healthy controls with the aim of understanding the regulatory mechanisms of said stages. Expression values were obtained by means of qPCR. The most relevant miRNA were validated in an independent set of samples. In order to determine the effect of the miRNA pattern, the expression of some predicted target genes were studied by qPCR. Gene interaction networks were constructed in order to obtain a system biology and multivariate view of the experimental data. The data analysis and later validation reveal that some miRNAs, e.g., hsa-mir-18b, hsa-mir-493 and hsa-mir-599, specially hsa-mir-18b and hsa-mir-599, may be relevant at the time of relapse and that other miRNA (hsa-mir-96, hsa-mir-148a, hsa-mir-184, has-mir-30a-5p and hsa-mir-193, specially hsa-mir-96, may be involved in the remitting phenomena. This study highlights the importance of the miRNA expression in the molecular mechanisms implicated in the disease.

## 1. Materials and Methods

### Recruitment of individuals

All patients were recruited in the Neurology Department of Hospital Donostia, located in the region of Gipuzkoa (Basque Country, Spain). The study was approved by the local institutional review board and all the samples were obtained with the written informed consent of the subjects. The patients were diagnosed as having MS according to the McDonald Criteria [McDonald, W. et al. Recommended diagnostic criteria for multiple sclerosis: guidelines from the International Panel of the diagnosis of multiple sclerosis. Ann. Neurol. 50, 121-127 (2001); Poser, C.M. Revisions to the 2001 McDonald diagnostic criteria. Ann. Neurol. 59, 727-728 (2006)].

In a first group (Group A), 21 blood samples were obtained: 9 from patients in remission, 4 from patients during a relapse before the administration of steroids and 8 from healthy volunteers. Total RNA, including miRNA, was extracted from these samples to carry out the study.

Samples were collected from two other non-related groups to validate independently some of the results obtained:

- Group B: mRNA was obtained from 42 samples; 14 in remitting status, 13 in relapse status and 15 controls.
- Group C: miRNA was extracted from 14 samples; 4 in relapse, 3 in remitting status and 7 healthy controls.

Demographic data of the individuals are shown in Table 2.

Blood extraction was always performed in the early morning and RNA extraction was carried out no more than 2 hours after the blood was collected.

## Table 2

### Demographic data of individuals

| sample | Status | Stage | Sex | Age | | Tev | age at onset | EDSS | Relapse Symptoms | te |
|---|---|---|---|---|---|---|---|---|---|---|
| 1 | EM | Relapse | Male | 35 | | 1 | 34 | 3 | Sensitive | 2 |
| 2 | EM | Relapse | Male | 53 | | 10 | 43 | 4,5 | Paraparesy and muscle weakness in Right leg and inestability | 1 |
| 3 | EM | Relapse | Male | 38 | | 11 | 27 | 3,5 | brote medular | 1 |
| 4 | EM | Relapse | Female | 46 | 43 ± 8,1 | 19 | 27 | 5 | Spasticity in legs and dificulty in moving | 2 |
| 5 | CON | | Male | 33 | | | | | | |
| 6 | CON | | Female | 34 | | | | | | |
| 7 | CON | | Female | 28 | | | | | | |
| 8 | CON | | Female | 35 | | | | | | |
| 9 | CON | | Female | 32 | | | | | | |
| 10 | CON | | Female | 26 | | | | | | |
| 11 | CON | | Female | 28 | | | | | | |
| 12 | CON | | Male | 33 | 31,13 ± 3,3 | | | | | |
| 13 | EM | Remitting | Female | 45 | | 22 | 23 | 2,5 | | |
| 14 | EM | Remitting | Female | 37 | | 7 | 30 | 1,5 | | |
| 15 | EM | Remitting | Female | 54 | | 11 | 43 | 2 | | |
| 16 | EM | Remitting | Female | 45 | | 12 | 33 | 6 | | |
| 17 | EM | Remitting | Female | 69 | | 22 | 47 | 2 | | |
| 18 | EM | Remitting | Female | 41 | | 20 | 21 | 3,5 | | |
| 19 | EM | Remitting | Female | 38 | | 2 | 36 | 2 | | |
| 20 | EM | Remitting | Male | 33 | | 11 | 21 | 6 | | |
| 21 | EM | Remitting | Male | 45 | 45,2 ± 11 | 5 | 40 | 2 | | |

Clinical description of the patients. Tev: Time of evolution (years). EDSS: Expanded Disability Status Score. Te: Time from the relapse onset and the blood extraction (in days)

## RNA extraction, reverse transcription (RT) and quantitative PCR (qPCR)

Total RNA was extracted from blood using the *Ambion Leucolock* kit (AM1923) working with the alternative protocol so as to keep the small RNA fraction. The RNA obtained was quantified in triplicate using a NanoDrop spectrophotometer (NanoDrop Technologies, USA). A common bias in the interpretation of the miRNA profiles from whole blood may be introduced by the high concentration of miRNA from erythrocytes [Chen, S.Y., Wang, Y., Telen, M.J., & Chi, J.T. The genomic analysis of erythrocyte microRNA expression in sickle cell diseases. PLoS. ONE. 3, e2360 (2008)]. In this study, such a bias was avoided by isolating PBMC in a filter prior to RNA purification.

In the case of group B, the samples came from RNA samples that are being collected systematically by the inventors' group. They were extracted using a Versagene™ Kit (Gentra, Minneapolis, USA). This kind of extraction method entails the loss of the small molecules of RNA, i.e. miRNA.

The cDNA was synthesized from total RNA using a Multiplex RT for Taqman array kit (Applied Biosystems, Foster City, CA). Briefly, this kit consists of 8 predefined RT primer pools containing up to 48 RT primers each. Each of these 8 pools contains the same endogenous controls (RNU48). This technology has been developed to detect only full length mature miRNA but not their precursors or their partially-degraded products.

A qPCR was performed by using the Taqman® Low Density Array (TLDA) Human MicroRNA Panel v1.0 from Applied Biosystems. This TLDA included 365 miRNA assays plus an endogenous control. The qPCR was performed using an Applied Biosystems 7900 Sequence Detection System. Ct values were determined using the automatic threshold in *RQ manager v1.1* analysis software.

Two normalization steps were used: the first normalization consisted in loading the same quantity of template RNA and the second in normalizing the data against an endogenous gene. This endogenous control (RNU48) was chosen for this study as the least variable of all endogenous genes included in the TLDA assays. Consequently, data was normalized to RNU48, using the values of each of the 8 pools, i.e. each gene pool was normalized against the endogenous gene that was converted to cDNA in the same pool, to avoid introducing bias in the results.

Relative quantification of miRNA expression was calculated with the $2^{-ddCT}$ method (Applied Biosystems User Bulletin N° 2 (P/N 4303859)). Quality of the data and quantification was computed using Real-Time Statminer© software (www.integromics.com). This software performs a classical t-test between the groups (relapse, remitting and control) and corrects them using the Benjamini-Hochberg algorithm [Benjamini, Y. & Hochberg, Y. Controlling the false discovery rate: A practical and powerful approach to multiple testing. J. Roy. Statist. Soc. Ser B 57, 289-300 (1995)] with the False Discovery Rate (FDR) set at a value of 5%.

**Statistical data analysis**

A non-parametric analysis that complements the classical t-test analysis was performed trying to reveal alternative results over the low number of available samples. The expression patterns of the three groups was compared by pairs: relapse vs remitting, relapse vs control and remitting vs control. To accomplish this task, a non-parametric ranking method called Symmetrical Uncertainty (*SU*) sorts all the miRNA according to their statistical relevance over each of the three comparisons using the following coefficient:

$$SU(Y,C) = \frac{2(H(Y) - H(Y|C))}{H(Y) + H(C)}$$

wherein

*Y* is the predictive variable (in this case, each miRNA),
*C* is the class label to be predicted (depending on the comparison carried out, it takes two of the following three values: remitting, relapse and control),
*H(Y)* is the entropy of *Y* and *H(Y|C)* is the conditional entropy of *Y* given *C* [Cover TM & Thomas JA. Elements of Information Theory (Wiley Interscience, 2006)].

The SU ranking is based on the mutual information between each miRNA expression level and the phenotype label. Being a univariate coefficient, it measures the uncertainty reduction of the class variable C when the expression value of an miRNA (denoted as Y in the above formulation) is known. As the SU metric only deals with discrete/categorical variables, the DCT expression of each miRNA was firstly discretized into three intervals by means of an equal width discretization.

In order to get a system biology and multivariate view of the experimental data, the inventors undertook the construction of co-expression networks to investigate the possible regulations within two out of our three comparisons (relapse *vs* remitting and remitting *vs* control). For this purpose, the inventors borrowed a technique for building gene interaction networks [Armañanzas, R., Inza, I., & Larrañaga, P. Detecting reliable gene interactions by a hierarchy of Bayesian network classifiers. Computer methods and programs in biomedicine 91, 110-121 (2008)] and applied it to their DCT expression data. This algorithm makes use of three main components to find reliable dependences from data: a bootstrap re-sampling algorithm, a Bayesian network classifier and a dimensionality reduction technique. The algorithm's construction scheme is focused on finding highly reliable dependences from raw data. The bootstrap step re-samples the original data $B$ times, obtaining $B$ similar datasets. For each sampled dataset a dimensionality reduction step is made using the correlation-based filter selection approach (CFS) [Hall MA & Smith LA Feature subset selection: a correlation based filter approach. *Proceedings of the Fourth International Conference on Neural Information Processing and Intelligent Information Systems* 855-858 (1997)]. The CFS returns sets of relevant features that show a high degree of correlation with the class label while the redundancy degree among them is kept as low as possible. Each sampled dataset is projected to contain only the selected features and afterwards a $k$-dependence Bayesian network classifier is induced from that data [Sahami, M. Learning limited dependence Bayesian classifiers, in *Proceedings of the Second International Conference on Knowledge Discovery and Data Mining* 335-338, 1996]. All the probabilistic dependences found by the $B$ final classifier are stored.

The algorithm's output is a hierarchy of probabilistic dependences found during the whole process. When a cut-off threshold $T$ is set, it is possible to retrieve a graphical structure in which only those probabilistic conditional dependences that have been configured at least $T$ times are displayed. Each arc in the final structure is associated with a robustness value which reflects the number of times the arc is configured in the different bootstrap re-samplings.

A total of 10,000 re-samplings was performed with their correspondent CFS and $k$-DB data mining techniques. The value of $k$ in $k$-DB was set to four, keeping to the value suggested in the original work.

**Sequence of the miRNA genes**

The selected miRNA genes were amplified by PCR (primers sequences available on request) and the PCR product was sequenced in an ABI3130 automatic sequencer (Applied Biosystems) using Bigdye v3.1. The used primers were designed based on the mirbase [Griffiths-Jones, S. The microRNA Registry. Nucleic Acids Res. 32, D109-D111 (2004); Griffiths-Jones,S. miRBase: the microRNA sequence database. Methods Mol. Biol. 342, 129-138 (2006); Griffiths-Jones,S., Saini,H.K., van,D.S., & Enright,A.J. miRBase: tools for microRNA genomics. Nucleic Acids Res. 36, D154-D158 (2008)] sequence information and using the Generunner software (www.generunner.com). Group A samples (n=21) were analyzed as well as 40 healthy controls.

**Validation of the target genes**

An independent set of 42 samples (group B) was studied. The expression was analyzed by qPCR using SYBRgreen as fluorescent and pre-designed primers from geneglobe (www.geneglgbe.com). The assay codes can be found in Table 3. The data were analyzed using the same software and the same methodology described above, using as the endogenous gene GAPDH. The expression of four selected genes was studied. Table 3 presents the miRNAs that joint to these genes and the group in which it is expected to be down-regulated.

## Table 3

## Target genes studied with their gene ID, the miRNA that binds to the gene, the group in which these genes are expected to be down-regulated and the Geneglobe Assay code

|  | Gene ID | miRNA | Expected to be down regulated in: | Assay code |
|---|---|---|---|---|
| ARHGEF12 | 23365 | hsa-mir-96 / hsa-mir-148 / hsa-mir-193 | Remitting group | QT00006762 |
| CELSR2 | 1952 | hsa-mir-96 | Remitting group | QT00010948 |
| TAOK3 | 51347 | hsa-mir-599 | Relapse group | QT00059843 |
| GAB1 | 2549 | hsa-mir-18b | Relapse group | QT00014154 |

**Individual validation of the miRNA expression**

The validation of the expression of the selected miRNA genes was performed in an independent set of 14 samples (Group C). The qPCR was performed in a 7900 sequence detection system using pre-designed Taqman probes (Applied Biosystems).

**2. Results**

qPCR was used to study the expression of 364 miRNAs in samples from 4 MS patients during a relapse and from 9 patients during remission. Also 8 healthy controls were analyzed.

On average, 45% of the miRNA analyzed were expressed in any given sample. Although several miRNA reached nominal significance in the t-test, only three remained significant after correction for multiple testing (with an FDR threshold of 5%). The transcript hsa-mir-18b showed increasing expression in the relapsing group when comparing the relapse group vs the control group (RQ: 52.1). The transcripts hsa-mir-493 and hsa-mir-599 showed expression in the relapsing group whereas they were not expressed in controls. These two miRNAs are also expressed in the remitting group but did not reach any statistical significance in the comparisons.

In order to complement the information of the classical statistical analysis, the symmetrical uncertainty (*SU*) correlation degree of each miRNA with respect to the class phenotype was calculated, providing a ranked list of all miRNAs. The top ten miRNA emerging from these rankings are shown in Table 4. The rankings have been made for three different comparisons; relapse versus remitting, remitting versus controls and relapse versus controls. Highlighted are the significant miRNAs found in the previous analysis.

**Table 4**

| Top 10 genes from the *SU* analysis for each of the three comparisons | | | | | | |
|---|---|---|---|---|---|---|
| | | Relevance as Symmetrical Uncertainty | | | | |
| | | Relap vs. Rem | | Rem vs. CON | | Relap vs. CON | |
| | | gene | SU | gene | SU | gene | SU |
| | 1 | hsa-miR-542-5p | 0.5277 | hsa-miR-96 | 0.4832 | hsa-miR-599 | 0.8651 |
| | 2 | hsa-miR-376a | 0.4921 | hsa-miR-30a-5p | 0.2989 | hsa-miR-18b | 0.6416 |
| | 3 | hsa-miR-18b | 0.4048 | hsa-miR-30e-5p | 0.2959 | hsa-miR-423 | 0.6367 |
| | 4 | hsa-miR-34c | 0.4039 | hsa-miR-599 | 0.2959 | hsa-miR-125b | 0.5738 |
| | 5 | hsa-miR-489 | 0.4039 | hsa-miR-193a | 0.2959 | hsa-miR-383 | 0.5392 |
| | 6 | hsa-miR-554 | 0.4039 | hsa-miR-337 | 0.2959 | hsa-miR-509 | 0.5392 |
| | 7 | hsa-miR-600 | 0.4039 | hsa-miR-449b | 0.2591 | hsa-miR-30e-5p | 0.5392 |
| | 8 | hsa-miR-652 | 0.4039 | hsa-miR-184 | 0.2477 | hsa-miR-487b | 0.5167 |
| | 9 | hsa-miR-214 | 0.3863 | hsa-miR-328 | 0.2283 | hsa-miR-222 | 0.4970 |
| | 10 | hsa-miR-328 | 0.3729 | hsa-miR-146b | 0.2238 | hsa-miR-127 | 0.4965 |

[Highlighted miRNAs (has-mir-18b and has-mir-599) were also significant in the corrected t-test]

A system biology analysis was performed to obtain information about the relationships between the different miRNA. Interaction networks were built in two groups:

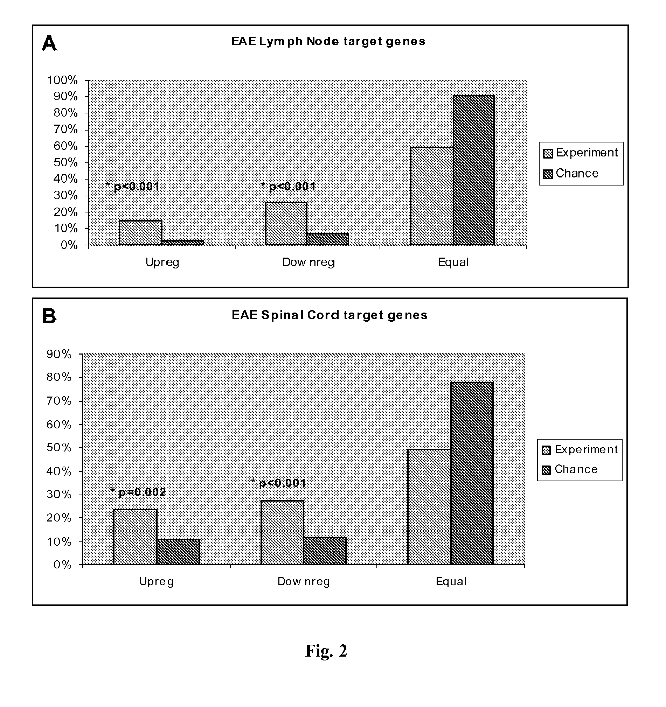

- relapse versus remitting, in order to obtain information about the relapse phenomena in the patients; and
- remitting versus controls, in order to obtain information about the MS (Fig 2 A-B).

From these analyses of the expression data, 8 miRNAs were selected in which further analysis were performed:

- three miRNAs, hsa-mir-18b, hsa-mir-493 and hsa-mir-599, were choosen because they reached the significance level in the corrected t-test used to compare relapse status with control samples; and
- five miRNAs, hsa-mir-96, hsa-mir-148a, hsa-mir-184, hsa-mir-30a-5p, and hsa-mir-193, were selected, coming from the network analysis that differentiates between remitting and control groups. From this network, inventors selected the two miRNAs with more interactions (hsa-mir-96 and has-mir-30a-5p), the two with arcs showing the highest robustness values (hsa-mir-184 with 1557 and hsa-mir-193a with 1358) and the other parent of hsa-mir-96, the hsa-mir-184.

**Validation of the miRNA expression**

To validate these results, the following three miRNA: hsa-mir-18b, hsa-mir-96 and hsa-mir-599, were studied in an independent set of samples (group C).
The has-mir-18b and has-mir-599 were up-regulated four and five times more in the relapse group than in the controls. For has-mir-96 no differences in the expression between the groups were obtained.

**Sequencing of the miRNA genes**

The gene sequence of 3 (hsa-mir-96, hsa-mir-493 and hsa-mir-18b) of the 7 selected mature miRNAs was amplified and sequenced in the Group A samples and in a control group (n=40). Said three miRNAs were selected as representative of the comparisons: hsa-mir-96 in the remitting versus control study and the other two in the relapse versus remitting comparison. No polymorphisms were associated with any of the groups.

**miRNA targets**

In order to provide a biological interpretation of our findings, the predicted targets of each relevant miRNA was searched in three different databases; **mirbase targets v5, Targetsan v4.2** [Lewis, B.P., Shih, I.H., Jones-Rhoades, M.W., Bartel, D.P., & Burge, C.B. Prediction of mammalian microRNA targets. Cell 115, 787-798 (2003); Lewis, B.P., Burge, C.B., & Bartel, D.P. Conserved seed pairing, often flanked by adenosines, indicates that thousands of human genes are microRNA targets. Cell 120, 15-20 (2005); Grimson, A. et al. MicroRNA targeting specificity in mammals: determinants beyond seed pairing. Mol. Cell 27, 91-105 (2007)] and **Pictar** [Krek, A. et al. Combinatorial microRNA target predictions. Nat. Genet. 37, 495-500 (2005)].

Table 5 lists the number of predicted targets for these miRNAs according to each database (two searches with different confidence thresholds were performed in **mirbase).**

**Table 5**

**Predicted targets for each miRNA in three databases**

| | mirbase | | Pictar | Targetscan | Common | |
|---|---|---|---|---|---|---|
| | $p<0.05$ | $p<0.005$ | | | | |
| 18 | 775 | 327 | 151 | 149 | 14 | *Relapse vs Control* |
| 599 | 783 | 185 | X | 173 | 11 | |
| 493 | 866 | 244 | X | 496 | 3 | |
| 96 | 909 | 361 | 698 | 592 | 57 | *Non Relapse vs Control* |
| 184 | 819 | 289 | 22 | 17 | 3 | |
| 148A | 918 | 353 | 429 | 434 | 46 | |
| 193 | 819 | 362 | 134 | 208 | 14 | |

[The column labeled as "common" represents the common predicted targets in the three databases]

Theoretically, these miRNAs should inhibit the expression of a certain number of target genes. The databases offer predicted information about the targets, but there are few experimental results to support it. In our analysis we took a conservative approach, taking as target genes only the common results from the three different prediction algorithms (in the **mirbase** case we selected the $p<0.005$ column).

To validate these results, the expression in blood of four of these targets in an independent sample set (GroupB) was checked, obtaining no statistical differences in the expression pattern.

Since miRNAs are highly conserved across species [Weber, M.J. New human and mouse microRNA genes found by homology search. FEBS J. 272, 59-73 (2005); Ibañez-Ventoso, C., Vora, M., & Driscoll, M. Sequence relationships among C. elegans, D. melanogaster and human microRNAs highlight the extensive conservation of microRNAs in biology. PLoS. ONE. 3, e2818 (2008)], the murine EAE model was used to validate our findings. To this end, a large multi-tissue, longitudinal gene expression profiling dataset in mouse EAE Lymph Node [Otaegui, D. et al. Increased transcriptional activity of milk-related genes following the active phase of experimental autoimmune encephalomyelitis and multiple sclerosis. J. Immunol. 179, 4074-4082 (2007)] and spinal cord [Baranzini, S.E., Bernard, C.C., & Oksenberg, J.R. Modular transcriptional activity characterizes the initiation and progression of autoimmune encephalomyelitis. J. Immunol. 174, 7412-7422 (2005)] was mined, focusing on targets of the same miRNAs the inventors identified in their cohort of MS patients. In order to check whether said target selected genes were really related with the disease, a group of 11 miRNAs was randomly selected from those that were not differentially expressed in the first analysis.

The expression of said target genes was checked for each miRNA-gene in the disease moment (at the peak of the disease and after it) between the control group and the EAE group. The target genes were classified in four groups: not found in the dataset, up-regulated, down-regulated and equally-expressed.

The results of the found genes are summarized as percentages in Figure 2. A chi-square analysis has been performed between the groups. The figure shows the results of the analysis for the selected genes (clear) and for the randomly selected group (dark). The analyzed target-genes were differentially distributed ($p<0.001$) between experimental and random group in the up-regulated and down-regulated class.

A pathway analysis was conducted in Panther [Mi, H., Guo, N., Kejariwal, A., & Thomas, P. PANTHER version 6: protein sequence and function evolution data with expanded representation of biological pathways. Nucleic Acids Res. 37, D247-D252 (2007)] database for all miRNA (experimental and random). Out of the eight miRNAs targets, only targets of hsa-mir-96 appeared significantly enriched in 8 pathways (Table 6).

**Table 6**

| Pathway analysis of the hsa-mir-96 targets | | | | | |
|---|---|---|---|---|---|
| Pathway_hsa-miR-96 targets | NCBI | 96 | expected | ratio | P-value |
| Muscarinic acetylcholine receptor 1 and 3 signaling pathway | 62 | 5 | 0,14 | 35,7 | 5,39E-05 |
| Alpha adrenergic receptor signaling pathway | 29 | 3 | 0,06 | 50,0 | 6,88E-03 |
| Unclassified | 22436 | 39 | 50,29 | 0,8 | 1,01E-02 |
| Endothelin signaling pathway | 98 | 4 | 0,22 | 18,2 | 1,23E-02 |
| Interleukin signaling pathway | 194 | 5 | 0,43 | 11,6 | 1,29E-02 |
| Wnt signaling pathway | 348 | 6 | 0,78 | 7,7 | 2,18E-02 |
| Histamine H1 receptor mediated signaling pathway | 43 | 3 | 0,1 | 30,0 | 2,19E-02 |
| Metabotropic glutamate receptor group I pathway | 44 | 3 | 0,1 | 30,0 | 2,35E-02 |
| Angiotensin II-stimulated signaling through G proteins and beta-arrestin | 53 | 3 | 0,12 | 25,0 | 4,04E-02 |

### 3. Discussion

From these data, three miRNA (hsa-mir-18b, hsa-mir-493 and hsa-mir-599) were identified showing differential expression in MS patients experiencing a relapse when compared to controls. Classic parametric tests did not detect differentially expressed miRNA between samples from patients in remission vs controls. However, a network-based approach identified 5 miRNAs (hsa-mir-96, hsa-mir-148a, hsa-mir-184, hsa-mir-30a-5p, and hsa-mir-193) that distinguished samples from these two phases of the disease.

Inventors hypothesized that if a given miRNA was over-expressed in a particular group of samples, the targets of this miRNA should be down-regulated. Said hypothesis was checked in an EAE model. Interestingly, in this model the target genes of all 8 differentially expressed miRNAs appeared significantly down-regulated more times in the targets selected by the inventors' experiment than in a random target list.

However, the same effect could be seen in the up-regulated genes. These could be a retroactive regulation of the mRNA that are regulated in a translational repression form.

A biological interpretation of miRNA function in MS is complicated by the fact that most of the miRNA targets are predicted from bioinformatics analysis and are not yet validated in biological studies. To enhance the confidence, inventors only worked with consensus targets from the three public miRNA databases.

The discussion has been structured around the groups analyzed, i.e. patients in relapse status, patients in remitting status and control samples.

### Patients in relapse status

The results from the t-test gave inventors three differentially expressed genes between relapse and control samples. It could be expected the same differences between relapse and remitting groups. When that was checked, hsa-mir-18b and hsa-mir-599, but not hsa-mir-493, were up-regulated in relapse, this being significant in the uncorrected p-values but not in the FDR corrected values.

In the relapse versus remitting network, two of these genes, hsa-mir-18b and hsa-mir-599 appeared correlated with a direct probabilistic relationship. Moreover, in the SU analysis, hsa-mir-18b appears in the third position in the relapse-remitting ranking. The expressions of these two genes have been validated in an independent set of samples. Taken all together, these results highlight the importance of hsa-mir-18b and hsa-mir-599 in the mechanisms of the relapse. Their role remains unclear, but should be related with the regulation of the proposed target genes.

In the analyses of the pathways in which the target genes of these two miRNA may be implicated neither the target genes of each miRNA individually nor the target genes in common between both miRNA give significant results.

The study of the target genes showed no clear inhibition, as might be expected, maybe because regulation of the miRNA is occurring at the translational level rather than at the expression level.

These results support the idea that the expression of the miRNA could be useful as a biomarker of the relapse status.

**Patients in remitting status**

The proposed network obtained in the comparison between remitting status and control samples indicate four micro RNAs likely to be implicated in the relapse (hsa-mir-96, hsa-mir-148a, hsa-mir-184, hsa-mir-30a-5p and hsa-mir-193). The results strongly suggest that hsa-mir-96 is an important candidate for further studies. This gene is also first in the SU ranking when remitting and control groups are compared. Although the classic qPCR analysis of the expression of this gene gave no significant differences, in the data it could be appreciated that this gene is more expressed in remitting samples than in controls, and less in relapse samples than in remitting, so it seems to be characteristic of the remitting phase of the disease. In the validation with an independent set the results are the same: no differences between the groups but a similar trend in the data.

The Gene Ontology approach to the target-genes of hsa-mir-96 gave a list of 8 pathways that reached the significant level. As could be expected, within this list inventors found a classic immunologic associated pathway such as *Interleukin signaling pathways.* Two pathways, the *Metabotropic glutamate receptor group I pathway* and the *Muscarinic acetylcholine receptor I and 3 signaling pathway,* related with glutamate, are also present. Glutamate has been widely related with pathological mechanisms of the MS such as exocitotoxicity [Vallejo-Illarramendi,A., Domercq,M., Perez-Cerda,F., Ravid,R., & Matute,C. Increased expression and function of glutamate transporters in multiple sclerosis. Neurobiol. Dis. 21, 154-164 (2006); Matute,C. Interaction between glutamate signalling and immune attack in damaging oligodendrocytes. Neuron Glia Biol. 3, 281-285 (2007)]. These mechanisms are related with the CNS but could be being expressed in blood by the activated T-cells.

The *Wnt signaling pathway* is also present in the found pathways. The Wnt gene has been proposed as an important player in the development of effector T-cells and in the activation of the regulatory T-cell [Staal, F.J., Luis, T.C., & Tiemessen, M.M. WNT signalling in the immune system: WNT is spreading its wings. Nat. Rev. Immunol. 8, 581-593 (2008)]. All these pathways may be potential subjects for more in-depth studies, but at this point, their immunological role makes our data more reliable.

**MS and miRNA**

A relationship between miRNA expression and MS could be expected since some of the functions attributed to the miRNA include stress response, immunomodulation [de Yebenes,V. et al. miR-181b negatively regulates activation-induced cytidine deaminase in B cells. J. Exp. Med. (2008); Baltimore, D., Boldin, M.P., O'Connell, R.M., Rao, D.S., & Taganov, K.D. MicroRNAs: new regulators of immune cell development and function. Nat. Immunol. 9, 839-845 (2008)] and neuroprotection [Nelson, P.T., Wang, W.X., & Rajeev, B.W. MicroRNAs (miRNAs) in neurodegenerative diseases. Brain Pathol. 18, 130-138 (2008). Moreover, bioinformatics-based predictions propose that 30 % of the human genes are regulated by miRNAs [Ross,J.S., Carlson,J.A., & Brock,G. miRNA: the new gene silencer. Am. J. Clin. Pathol. 128, 830-836 (2007)]. Therefore, the inventors hypothesized that a sizeable proportion of the mRNA differentially expressed between samples from patients during a relapse and during remission ought to be regulated by miRNAs.

These results support the role of miRNA expression patterns in MS. The reliability of the data is supported by the different statistical approaches, by the validation in an independent cohort of samples and by the congruent results, both in the gene ontology analysis and in the animal model analysis.

Although this should be validated in a larger cohort of samples, at least one miRNA is outstanding as a potential biomarker in MS and at least two more show potential to be good targets for future biomarker studies to characterize the relapse status.

**Claims**

1. A method of diagnosing Multiple Sclerosis (MS) in a subject, which comprises comparing:

   a) the level of expression of a miRNA selected from the group consisting of hsa-mir-18b, hsa-mir-493, hsa-mir-599, hsa-mir-96, hsa-mir-148a, hsa-mir-184, hsa-mir-30a-5p, hsa-mir-193 and combinations thereof, in a sample from said subject; and

b) the normal level of expression of said miRNA(s) in a reference sample,

wherein a statistically significant increase in the level of expression of said miRNA in the subject sample with respect to the normal level of said miRNA(s) in the reference sample is indicative that the subject is afflicted with MS.

2. A method for assessing if a subject afflicted with Multiple Sclerosis (MS) is experiencing a relapse, which comprises comparing:

a) the level of expression of a miRNA selected from the group consisting of hsa-mir-18b, hsa-mir-493, hsa-mir-599, and combinations thereof, in a test sample from said subject; and

b) the normal level of expression of said miRNA(s) in a reference sample,

wherein a statistically significant increase in the level of expression of said miRNA in the subject sample with respect to the normal level of said miRNA(s) in the reference sample is indicative that the subject afflicted with MS is experiencing a relapse.

3. A method for assessing if a subject afflicted with Multiple Sclerosis (MS) is experiencing a remission, which comprises comparing:

a) the level of expression of a miRNA selected from the group consisting of hsa-mir-96, hsa-mir-148a, hsa-mir-184, hsa-mir-30a-5p, hsa-mir-193, and combinations thereof, in a test sample from said subject; and

b) the normal level of expression of said miRNA(s) in a reference sample,

wherein a statistically significant increase in the level of expression of said miRNA(s) in the subject sample with respect to the normal level of said miRNA(s) in the reference sample is indicative that the subject afflicted with MS is experiencing a remission.

4. A method for assessing if a subject afflicted with Multiple Sclerosis (MS) is experiencing a remission, which comprises comparing:

a) the level of expression of a miRNA selected from the group consisting of hsa-mir-18b, hsa-mir-493, hsa-mir-599, and combinations thereof, in a test sample from said subject; and

b) the level of expression of said miRNA(s) in a control sample, said control sample being a sample from the same subject under analysis obtained during a period in which said subject afflicted with MS under analysis is experiencing a relapse,

wherein a statistically significant decrease in the level of expression of said miRNA(s) in the subject sample with respect to the level of said miRNA(s) in the control sample is indicative that the subject afflicted with MS is experiencing a remission.

5. A method for assessing if a subject afflicted with Multiple Sclerosis (MS) is experiencing a relapse, which comprises comparing:

a) the level of expression of a miRNA selected from the group consisting of hsa-mir-96, hsa-mir-148a, hsa-mir-184, hsa-mir-30a-5p, hsa-mir-193, and combinations thereof, in a test sample from said subject; and

b) the level of expression of said miRNA(s) in a control sample, said control sample being a sample from the same subject under analysis obtained during a period in which said subject afflicted with MS under analysis is experiencing a remission,

wherein a statistically significant decrease in the level of expression of said miRNA(s) in the subject sample with respect to the level of said miRNA(s) in the control sample is indicative that the subject afflicted with MS is experiencing a relapse.

6. Method according to anyone of claims 1 to 5, wherein said sample comprises blood mononuclear cells.

7. Method according to anyone of claims 1 to 5, wherein the level of expression of said miRNAs is determined by multiplex and/or singleplex real-time RT-PCR; or by single-molecule detection; or by a bead-based flow cytometric method; or by an assays using arrays of nucleic acids.

8. Method according to claim 7, wherein the level of expression of said miRNAs is determined by real-time quantitative RT-PCR (qRT-PCT).

9. Method according to claim 1, wherein said miRNA is selected from the group consisting of hsa-mir-18b, hsa-mir-493, hsa-mir-599 and combinations thereof.

10. Method according to claim 1, wherein said miRNA is selected from the group consisting of hsa-mir-96, hsa-mir-148a, hsa-mir-184, hsa-mir-30a-5p, hsa-mir-193 and combinations thereof.

11. A method of designing a therapy for a subject afflicted with Multiple Sclerosis (MS), which comprises:

- determining if a subject afflicted with MS is experiencing a relapse according to the method of claims 2 or 5, and selecting a drug suitable for treatment of MS in a relapse status; or, alternatively,
- determining if a subject afflicted with MS is experiencing a remission according to the method of claims 3 or 4, and selecting a drug suitable for treatment of MS in a remission status.

FIG. 1

**Fig. 2**

**EUROPEAN SEARCH REPORT**

Europäisches
Patentamt
European
Patent Office
Office européen
des brevets

Application Number

EP 09 38 2108

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/118806 A2 (AMBION INC [US]; BROWN DAVID; CONRAD RICK; DEVROE ERIC; GOLDRICK MARIA) 15 December 2005 (2005-12-15)<br>* abstract; examples 18,33 *<br>* page 69, line 33 - page 69, line 38 *<br>----- | 1,3,5-8, 10-11 | INV.<br>C12Q1/68 |
| A | WO 2008/153692 A (BRIGHAM & WOMENS HOSPITAL) 18 December 2008 (2008-12-18)<br>* abstract; claims 4,11,19 *<br>* page 4, lines 17-22 *<br>----- | 1-11 | |
| A | WO 2009/079592 A (CALIFORNIA INST OF TECHNOLOGY) 25 June 2009 (2009-06-25)<br>* abstract *<br>* paragraphs [0018], [0033] *<br>----- | 1-11 | |
| A,D | NELSON P.T. ET AL.: "MicroRNAs (miRNAs) in neurodegenerative diseases"<br>BRAIN PATHOLOGY,<br>vol. 18, no. 1, January 2008 (2008-01), pages 130-138, XP002558931<br>ISSN: 1015-6305<br>* abstract *<br>----- | 1-11 | **TECHNICAL FIELDS SEARCHED (IPC)**<br><br>C12Q |
| A,D | BARTEL D.P.: "MicroRNAs: genomics, biogenesis, mechanism, and function"<br>CELL, CELL PRESS, CAMBRIDGE, NA, US,<br>vol. 116, no. 2,<br>23 January 2004 (2004-01-23), pages 281-297, XP002358495<br>ISSN: 0092-8674<br>* the whole document *<br>----- | 1-11 | |

-/--

The present search report has been drawn up for all claims

| Place of search | Date of completion of the search | Examiner |
|---|---|---|
| Munich | 16 December 2009 | Barz, Wolfgang |

EPO FORM 1503 03.82 (P04C01)

Europäisches
Patentamt

European
Patent Office

Office européen
des brevets

# EUROPEAN SEARCH REPORT

Application Number

EP 09 38 2108

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JEYASEELAN K. ET AL.: "MicroRNAs as therapeutic targets in human diseases" EXPERT OPINION ON THERAPEUTIC TARGETS, ASHLEY PUBLICATIONS, LONDON, GB, vol. 11, no. 8, 1 August 2007 (2007-08-01), pages 1119-1129, XP009095263 ISSN: 1472-8222 * the whole document * | 1-11 | |
| T | OTAEGUI D. ET AL.: "Differential Micro RNA Expression in PBMC from Multiple Sclerosis Patients" PLOS ONE, vol. 4, no. 7, July 2009 (2009-07), page Article No.: e6309, XP002558932 ISSN: 1932-6203 * the whole document * | 1-11 | |

TECHNICAL FIELDS SEARCHED (IPC)

The present search report has been drawn up for all claims

| Place of search | Date of completion of the search | Examiner |
|---|---|---|
| Munich | 16 December 2009 | Barz, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&  : member of the same patent family, corresponding document

EPO FORM 1503 03.82 (P04C01)

**EP 2 290 102 A1**

## ANNEX TO THE EUROPEAN SEARCH REPORT
## ON EUROPEAN PATENT APPLICATION NO.

EP 09 38 2108

This annex lists the patent family members relating to the patent documents cited in the above-mentioned European search report.
The members are as contained in the European Patent Office EDP file on
The European Patent Office is in no way liable for these particulars which are merely given for the purpose of information.

16-12-2009

| Patent document cited in search report | | Publication date | Patent family member(s) | | Publication date |
|---|---|---|---|---|---|
| WO 2005118806 | A2 | 15-12-2005 | AU 2005250432 A1 | | 15-12-2005 |
| | | | CA 2572450 A1 | | 15-12-2005 |
| | | | EP 1771563 A2 | | 11-04-2007 |
| | | | EP 2065466 A2 | | 03-06-2009 |
| | | | JP 2008500837 T | | 17-01-2008 |
| WO 2008153692 | A | 18-12-2008 | NONE | | |
| WO 2009079592 | A | 25-06-2009 | US 2009203136 A1 | | 13-08-2009 |

EPO FORM P0459

For more details about this annex : see Official Journal of the European Patent Office, No. 12/82

26

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008147974 A
- WO 2009009457 A
- WO 2008153692 A
- US 5928907 A
- US 6015674 A
- US 6355420 B
- US 6916661 B
- US 6632526 B
- US 6524793 B
- US 6057134 A

- US 6891032 B
- US 7122303 B
- US 6458583 B
- US 6465183 B
- US 6461816 B
- US 7026124 B
- US 7052841 B
- US 7060809 B
- US 6436640 B

### Non-patent literature cited in the description

- **Oksenberg, J.R. ; Barcellos, L.F.** Multiple sclerosis genetics: leaving no stone unturned. *Genes Immun.,* 2005, vol. 6, 375-387
- **Alcina, A. et al.** IL2RA/CD25 gene polymorphisms: uneven association with multiple sclerosis (MS) and type 1 diabetes (T1D). *PLoS. ONE,* 2009, vol. 4, e4137
- **Gregory, S.G. et al.** Interleukin 7 receptor alpha chain (IL7R) shows allelic and functional association with multiple sclerosis. *Nat. Genet.,* 2007, vol. 39, 1083-1091
- **Achiron, A. ; Gurevich, M. ; Friedman, N. ; Kaminski, N. ; Mandel, M.** Blood transcriptional signatures of multiple sclerosis: unique gene expression of disease activity. *Ann. Neurol.,* 2004, vol. 55, 410-417
- **Baranzini, S.E. et al.** Transcription-based prediction of response to IFNbeta using supervised computational methods. *PLoS. Biol.,* 2005, vol. 3, e2
- **Ramanathan, M. et al.** In vivo gene expression revealed by cDNA arrays: the pattern in relapsing-remitting multiple sclerosis patients compared with normal subjects. *J. Neuroimmunol.,* 2001, vol. 116, 213-219
- **Otaegui, D. et al.** Increased transcriptional activity of milk-related genes following the active phase of experimental autoimmune encephalomyelitis and multiple sclerosis. *J. Immunol.,* 2007, vol. 179, 4074-4082
- **Satoh, J. ; Misawa, T. ; Tabunoki, H. ; Yamamura, T.** Molecular network analysis of T-cell transcriptome suggests aberrant regulation of gene expression by NF-kappaB as a biomarker for relapse of multiple sclerosis. *Dis. Markers,* 2008, vol. 25, 27-35

- **Bartel, D.P.** MicroRNAs: genomics, biogenesis, mechanism, and function. *Cell,* 2004, vol. 116, 281-297
- **Griffiths-Jones, S. et al.** miRBase: microRNA sequences, targets and gene nomenclature. *Nucleic Acids Res.,* 2006, vol. 34, D140-D144
- **Griffiths-Jones,S.** The microRNA Registry. *Nucleic Acids Res.,* 2004, vol. 32, D109-D111
- **Lewis, B.P. ; Burge, C.B. ; Bartel, D.P.** Conserved seed pairing, often flanked by adenosines, indicates that thousands of human genes are microRNA targets. *Cell,* 2005, vol. 120, 15-20
- **Baltimore, D. ; Boldin, M.P. ; O'Connell, R.M. ; Rao, D.S. ; Taganov, K.D.** MicroRNAs: new regulators of immune cell development and function. *Nat. Immunol.,* 2008, vol. 9, 839-845
- **O'Connell, R.M. ; Taganov, K.D. ; Boldin, M.P. ; Cheng, G. ; Baltimore, D.** MicroRNA-155 is induced during the macrophage inflammatory response. *Proc. Natl. Acad. Sci. U. S. A.,* 2007, vol. 104, 1604-1609
- **Nelson, P.T. ; Wang, W.X. ; Rajeev, B.W.** MicroRNAs (miRNAs) in neurodegenerative diseases. *Brain Pathol.,* 2008, vol. 18, 130-138
- **Yaguang X et al.** *Clin. Cancer Res,* 2006, vol. 12 (7), 2014-2024
- **Polman CH ; Reingold SC ; Edan G ; Filippi M ; Hartung HP ; Kappos L ; Lublin FD ; Metz LM ; McFarland HF ; O'Connor PW.** Diagnostic criteria for multiple sclerosis: 2005. *McDonald Criteria Ann Neurol.,* December 2005, vol. 58 (6), 840-6
- **Dowdy ; Wearden.** Statistics for Research. John Wiley & Sons, 1983
- **Neely et al.** *Nat. Methods,* 2006, vol. 3 (1), 41-6

- **Lu et al.** *Nature,* 2005, vol. 435, 7043
- **Nelson et al.** *Nat. Methods,* 2004, vol. 1 (2), 155-61
- **Wu et al.** *RNA,* 2007, vol. 13 (1), 151-9
- **McDonald, W. et al.** Recommended diagnostic criteria for multiple sclerosis: guidelines from the International Panel of the diagnosis of multiple sclerosis. *Ann. Neurol.,* 2001, vol. 50, 121-127
- **Poser, C.M.** Revisions to the 2001 McDonald diagnostic criteria. *Ann. Neurol.,* 2006, vol. 59, 727-728
- **Chen, S.Y. ; Wang, Y. ; Telen, M.J. ; Chi, J.T.** The genomic analysis of erythrocyte microRNA expression in sickle cell diseases. *PLoS. ONE,* 2008, vol. 3, e2360
- **Benjamini, Y. ; Hochberg, Y.** Controlling the false discovery rate: A practical and powerful approach to multiple testing. *J. Roy. Statist. Soc. Ser,* 1995, vol. B 57, 289-300
- **Cover TM ; Thomas JA.** Elements of Information Theory. Wiley Interscience, 2006
- **Armañanzas, R. ; Inza, I. ; Larrañaga, P.** Detecting reliable gene interactions by a hierarchy of Bayesian network classifiers. *Computer methods and programs in biomedicine,* 2008, vol. 91, 110-121
- **Griffiths-Jones, S.** The microRNA Registry. *Nucleic Acids Res.,* 2004, vol. 32, D109-D111
- **Griffiths-Jones,S.** miRBase: the microRNA sequence database. *Methods Mol. Biol.,* 2006, vol. 342, 129-138
- **Griffiths-Jones,S. ; Saini,H.K. ; van,D.S. ; Enright,A.J.** miRBase: tools for microRNA genomics. *Nucleic Acids Res.,* 2008, vol. 36, D154-D158
- **Lewis, B.P. ; Shih, I.H. ; Jones-Rhoades, M.W. ; Bartel, D.P. ; Burge, C.B.** Prediction of mammalian microRNA targets. *Cell,* 2003, vol. 115, 787-798
- **Grimson, A. et al.** MicroRNA targeting specificity in mammals: determinants beyond seed pairing. *Mol. Cell,* 2007, vol. 27, 91-105
- **Krek, A. et al.** Combinatorial microRNA target predictions. *Nat. Genet.,* 2005, vol. 37, 495-500
- **Weber, M.J.** New human and mouse microRNA genes found by homology search. *FEBS J.,* 2005, vol. 272, 59-73
- **Ibañez-Ventoso,C. ; Vora,M. ; Driscoll,M.** Sequence relationships among C. elegans, D. melanogaster and human microRNAs highlight the extensive conservation of microRNAs in biology. *PLoS. ONE,* 2008, vol. 3, e2818
- **Baranzini, S.E. ; Bernard, C.C. ; Oksenberg, J.R.** Modular transcriptional activity characterizes the initiation and progression of autoimmune encephalomyelitis. *J. Immunol.,* 2005, vol. 174, 7412-7422
- **Mi, H. ; Guo, N. ; Kejariwal, A. ; Thomas, P.** PANTHER version 6: protein sequence and function evolution data with expanded representation of biological pathways. *Nucleic Acids Res.,* 2007, vol. 37, D247-D252
- **Vallejo-Illarramendi,A. ; Domercq,M. ; Perez-Cerda,F. ; Ravid,R. ; Matute,C.** Increased expression and function of glutamate transporters in multiple sclerosis. *Neurobiol. Dis.,* 2006, vol. 21, 154-164
- **Matute,C.** Interaction between glutamate signalling and immune attack in damaging oligodendrocytes. *Neuron Glia Biol.,* 2007, vol. 3, 281-285
- **Staal, F.J. ; Luis, T.C. ; Tiemessen, M.M.** WNT signalling in the immune system: WNT is spreading its wings. *Nat. Rev. Immunol.,* 2008, vol. 8, 581-593
- **de Yebenes,V. et al.** miR-181b negatively regulates activation-induced cytidine deaminase in B cells. *J. Exp. Med.,* 2008
- **Ross,J.S. ; Carlson,J.A. ; Brock,G.** miRNA: the new gene silencer. *Am. J. Clin. Pathol.,* 2007, vol. 128, 830-836